Europäisches Patentamt

European Patent Office

Office européen des brevets

(11) Veröffentlichungsnummer: **0 209 732**

**A1**

(19)

(12) # EUROPÄISCHE PATENTANMELDUNG

(21) Anmeldenummer: 86108394.7

(22) Anmeldetag: 19.06.86

(51) Int. Cl.⁴: **B01J 23/78** , B01J 27/10 ,
//C07C17/156

(30) Priorität: 22.06.85 DE 3522471

(43) Veröffentlichungstag der Anmeldung:
28.01.87 Patentblatt 87/05

(84) Benannte Vertragsstaaten:
BE DE NL SE

(71) Anmelder: BASF Aktiengesellschaft
Carl-Bosch-Strasse 38
D-6700 Ludwigshafen(DE)

(72) Erfinder: Eichhorn, Hans-Dieter, Dr.
Buchnerstrasse 13
D-6700 Ludwigshafen(DE)
Erfinder: Mross, Wolf Dieter, Dr.
Anselm-Feuerbach-Strasse 21
D-6710 Frankenthal(DE)
Erfinder: Schwarzmann, Matthias, Dr.
Carl-Bosch-Strasse 54
D-6703 Limburgerhof(DE)
Erfinder: Becker, Hans-Jürgen, Dr.
Bergsteinstrasse 36
D-6730 Neustadt(DE)
Erfinder: Cordemans, Luc, Dr.
Jozef Mulslaan 13
B-2080 Kapellen(BE)

(54) Verfahren zur Herstellung eines geformten Trägerkatalysators, nach diesem Verfahren erhältlicher Trägerkatalysator sowie dessen Verwendung als Oxichlorierungskatalysator.

(57) Es wird vorgeschlagen ein Verfahren zur Herstellung eines geformten, Kupfer-(II)-chlorid und/oder Kupferoxichlorid enthaltenden Trägerkatalysators, dadurch gekennzeichnet, daß man

(a) zuerst die Oberfläche eines porösen Trägermaterials mit 2 bis 15 Gew.-% Kupferionen in Form von Kupfer-(II)-chlorid oder Kupferoxichlorid und 0 bis 5 Gew.-% Alkalimetallionen, jeweils bezogen auf das Gesamtgewicht des Katalysators, imprägniert und

(b) das so imprägnierte Trägermaterial zu Katalysatorformkörpern formt.

Der Katalysator ist insbesondere für Oxichlorierungsreaktionen geeignet.

**Verfahren zur Herstellung eines geformten Trägerkatalysators, nach diesem Verfahren erhältlicher Trägerkatalysator sowie dessen Verwendung als Oxichlorierungskatalysator**

Die Erfindung betrifft ein Verfahren zur Herstellung eines geformten, Kupfer-(II)-chlorid oder Kupferoxichlorid enthaltenden Trägerkatalysators, den nach diesem Verfahren erhältlichen Katalysator und dessen Verwendung als Oxichlorierungskatalysator im Katalysatorfestbett.

Die Oxichlorierung von Ethylen ist ein bekanntes, großtechnisch ausgeübtes Verfahren. Es haben sich insbesondere zwei Verfahren in der Technik eingeführt, bei denen man den Katalysator als Festbett anordnet, oder die Reaktion im Fließbett betreibt. Bei diesen Verfahren finden Katalysatoren Verwendung, die im wesentlichen Kupfer-(II)-chlorid und Promotoren wie Alkalimetallchloride, bevorzugt Kaliumchlorid, auf Trägern wie Aluminiumoxid, enthalten.

Verfahren zur Herstellung der genannten Katalysatoren für die Durchführung der Oxichlorierungsreaktion im Festbett sind zum Beispiel in den GB-B-11 04 666, DE-C-23 56 549 sowie DE-A-31 13 179 beschrieben.

Nach diesem Stand der Technik werden Träger aus beispielsweise Tonerde, Silikagel, Kieselsäure, Diatomenerde, Silikate, Aluminiumsilikate, bevorzugt jedoch α-Aluminiumoxid, in Form von Stücken mit einem Durchmesser zwischen 1 und 20 mm, z.B. als Tabletten, Kugeln, Ringe und andere Formkörper mit wäßrigen Lösungen getränkt, die Kupfer-(II)-chlorid und Zusätze wie Alkalimetallchloride enthalten. Nach dem Tränken werden die imprägnierten Formkörper getrocknet, in der Regel bei Temperaturen bis zu 300°C.

Alternativ wurde in der DE-A-31 13 179 vorgeschlagen, Mischungen aus der katalytisch wirksamen Komponente (Kupferchlorid) und dem Träger herzustellen und durch Tablettieren in die gewünschte Form zu bringen.

Bei der Durchführung des Oxichlorierungsverfahrens unter Verwendung von Katalysatoren, die nach dem Stand der Technik hergestellt wurden, ist bekannt, daß die Leistung einer Anlage im Laufe der Zeit immer geringer wird. Diese Leistungsverminderung kommt daher, daß die genannten Katalysatoren im Laufe der Zeit ihre Form verändern und allmählich zu Staub zerfallen. Dieser Staub setzt dem durchströmenden Gas einen höheren Widerstand entgegen als die ursprünglichen, größer dimensionierten Katalysatoren, so daß ein höheres Druckgefälle zwischen dem Reaktoreingang und dem Reaktorausgang überwunden werden muß. Da aber diese Druckdifferenz begrenzt ist, muß schließlich der Gasdurchfluß herabgesetzt werden, was zu einer Leistungsverminderung führt.

Üblicherweise ordnet man bei Oxichlorierungsverfahren, die ein-oder auch mehrstufig durchgeführt werden können, am Anfang einer Reaktionszone einen weniger aktiven Katalysator an und erhöht dann die Aktivität des Katalysators stufenweise oder auch kontinuierlich mit der Strömungsrichtung. Auf diese Weise können "hot spots" in der Katalysatorschüttung auf ein erträgliches Maß begrenzt werden, was für die Produktausbeuten und die Standzeit des Katalysators von großem Vorteil sind.

Verfahren zur Herstellung von Katalysatoren mit erniedrigter Aktivität sind bekannt. Es besteht die Möglichkeit, niedrigere Konzentrationen an Kupfer-(II)-chlorid auf dem Träger aufzubringen, oder den Katalysator mit Alkalimetallsalzen, bevorzugt mit Kaliumchlorid, zu dotieren. Eine andere Möglichkeit zur Herstellung von Katalysatoren mit erniedrigter Aktivität besteht darin, den Katalysator im Reaktor mit Inertmaterial zu verdünnen.

Während am Reaktoreingang also am günstigsten Katalysatorzonen mit niedriger Aktivität verwendet werden sollten, muß die Aktivität des Katalysators in Produktstromrichtung ansteigen und am Reaktorende, wo aufgrund der nur noch niedrigen Reaktantenpartialdrücke die Gefahr von Temperaturspitzen nur noch sehr gering ist, am höchsten sein, damit ein möglichst hoher Umsatz der Einsatzstoffe gewährleistet ist.

Der Erfindung liegt die Aufgabe zugrunde, ein Verfahren zur Herstellung eines geformten, Kupfer-(II)-chlorid oder Kupferoxichlorid enthaltenden Trägerkatalysators, sowie einen nach diesem Verfahren erhältlichen Katalysator bereitzustellen, der im Vergleich zu den Katalysatoren nach dem Stand der Technik eine höhere Standzeit und/oder eine bessere Aktivität und Selektivität aufweist.

Diese Aufgabe wird gelöst mit einem Verfahren der zuvor genannten Art, das dadurch gekennzeichnet ist, daß man

a) zuerst die Oberfläche eines porösen Trägermaterials mit 2 bis 15 Gew.-% Kupfer in Form von Kupfer-(II)-chlorid und/oder Kupferoxichlorid und 0 bis 5 Gew.-% Alkalimetallionen, jeweils bezogen auf das Gesamtgewicht des Katalysators, imprägniert und

b) das so imprägnierte Trägermaterial zu Katalysatorformkörpern formt.

Der nach diesem Verfahren erhältliche Katalysator weist überraschend eine verbesserte Standzeit und/oder Aktivität bzw. Selektivität im Vergleich zu Katalysatoren des Standes der Technik auf, die in bekannter Weise dadurch hergestellt werden, daß zunächst das Trägermaterial zu Formkörpern geformt und anschließend mit den Aktivkomponenten imprägniert wird. Die Ursache für die wesentliche Verbesserung der Katalysatoreigenschaften durch Vertauschen der Verformungs-und Imprägnierungsstufe ist nicht bekannt. Überraschend ist, daß sich die Vertauschung dieser Maßnahmen in einer derartigen Verbesserung der Katalysatoreigenschaften auswirkt.

Nach einer bevorzugten Ausführungsform des erfindungsgemäßen Verfahrens verwendet man 3 bis 10 Gew.-% Kupfer in Form von Kupfer-(II)-chlorid und/oder Kupferoxichlorid. Es ist ebenfalls bevorzugt, 0,2 bis 3 Gew.-% Alkalimetallionen, insbesondere Alkalichloride, zu verwenden. In Betracht kommen sämtliche Alkalimetallionen, insbesondere die Chloride, beispielsweise Lithium-, Natrium-, Kalium-, Rubidium-und Caesiumchlorid, besonders bevorzugt Kaliumchlorid.

Nach einer vorteilhaften Ausgestaltung des erfindungsgemäßen Verfahrens kann man das imprägnierte Trägermaterial mit Wasser anteigen und, gegebenenfalls unter Zusatz eines Extrudierhilfsmittels, zu Formkörpern extrudieren.

Nach einer anderen bevorzugten Ausführungsform des erfindungsgemäßen Verfahrens wird das imprägnierte Trägermaterial, gegebenenfalls unter Zusatz von Gleit-und/oder Bindemitteln, tablettiert.

Eine weitere, zweckmäßige Ausgestaltung des erfindungsgemäßen Verfahrens besteht darin, daß man das imprägnierte Trägermaterial, gegebenenfalls unter Zusatz von Granulierhilfsmitteln, granuliert.

Die Erfindung betrifft auch einen geformten Trägerkatalysator mit einem Gehalt an Kupfer-(II)-chlorid oder Kupferoxichlorid, der dadurch erhältlich ist, daß man

a) zuerst die Oberfläche eines porösen Trägermaterials mit 2 bis 15 Gew.-% Kupfer in Form von Kupfer-(II)-chlorid oder Kupferoxichlorid und 0 bis 5 Gew.-% Alkalimetallionen, jeweils bezogen auf das Gesamtgewicht des Katalysators, imprägniert und

b) das so imprägnierte Trägermaterial zu Katalysatorformkörpern formt.

Der erfindungsgemäß erhältliche Katalysator ist insbesondere geeignet für Oxichlorierungsreaktionen. Die Erfindung betrifft somit auch die Verwendung des Katalysators als Oxichlorierungskatalysator im Katalysatorfestbett.

Als Trägermaterial können beim erfindungsgemäßen Herstellungsverfahren die üblichen Träger wie Kieselgur, Silikagel, Diatomeenerde, Bims, Tonerden, Kieselsäure, Silikate und ähnliches Material verwendet werden. Bevorzugt wird als Trägermaterial Tonerde, insbesondere in Form von γ-Aluminiumoxid, eingesetzt.

Die Trägerpartikel weisen im allgemeinen eine BET-Oberfläche von ca. 30 bis ca. 350 m² auf, ihr Porenvolumen liegt etwa zwischen 0,3 bis 1 cm³/g. Bezüglich der Größenverteilung der Träger-Partikel erweist es sich als günstig, wenn ein großer Teil der Partikel eine Größe von weniger als 0,5 mm aufweist. Partikel mit einer Größe von mehr als 1 mm sollten nach Möglichkeit nicht vorhanden sein und deshalb vor dem Verformungsschritt abgetrennt und eventuell zu kleineren Partikeln gebrochen werden. Die Partikel können jedoch auch ohne Nachteil wesentlich kleiner sein; so darf z.B. auch ein großer Teil der Partikel eine Größe von weniger als 0,1 mm aufweisen.

Das Imprägnieren des Trägers mit den katalytisch wirksamen Komponenten kann nach den üblichen Techniken erfolgen. Zur Tränkung des Trägers können also wäßrige oder auch andere, z.B. alkoholische Lösungen, von Chloriden und/oder Oxichloriden des Kupfers verwendet werden.

Im allgemeinen wird der Träger mit wäßrigen Kupfer-(II)-chlorid-Lösungen getränkt. Die Lösung kann noch zusätzlich eine oder mehrere Alkaliverbindungen enthalten, z.B. Kaliumchlorid, Natriumchlorid, Lithiumchlorid, Rubidiumchlorid und/oder Caesiumchlorid, bevorzugt Kaliumchlorid, in einem molekularen Verhältnis von Kupferionen zu Kaliumionen von 1:1 bis 10:1. Es können aber auch andere Zusätze in der Tränklösung, z.B. Chlorwasserstoff, enthalten sein. Die Konzentration bzw. die Menge an Kupferchlorid-Lösung richtet sich nach der gewünschten Konzentration des Kupfers auf dem Träger. Beim Tränken des Trägers mit der Lösung aus Kupfer-(II)-chlorid und gegebenenfalls den Alkalimetallionenzusätzen entspricht die Gesamtmenge der Lösung in etwa der Wasseraufnahmefähigkeit des Trägers. Andererseits braucht sie aufgrund der kleinen Partikelgröße des Trägers nicht unbedingt der Wasseraufnahmefähigkeit des Trägers zu entsprechen. Überraschenderweise zeigt es sich, daß der Träger homogen und vollständig durchgetränkt wird, wenn auch weniger Lösung als der Wasseraufnahmefähigkeit des Trägers entspricht, verwendet wird. Ohne Nachteil kann die Gesamtmenge der Lösung auch bis auf 40 % der Wasseraufnahmefähigkeit des Trägers reduziert werden. .

Das Tränken des Trägers mit der Lösung wird zweckmäßigerweise in einer rotierenden Trommel vorgenommen. Dabei kann entweder der Träger vorgelegt und mit der Lösung besprüht, oder die Lösung vorgelegt und der Träger solange darin bewegt werden, bis die Lösung aufgesaugt ist. Die Temperatur beim Tränkvorgang soll vorzugsweise zwischen +10 und +70°C liegen.

Das Trocknen des imprägnierten Trägers erfolgt üblicherweise an Luft oder auch in Inertgasatmosphäre wie z.B. Stickstoff bei Temperaturen im Bereich von Raumtemperatur bis etwa 400°C, vorzugsweise durch langsames Erhitzen auf die Trocknungstemperatur und anschließendes mehrstündiges Tempern bei dieser Temperatur, wobei auch eine stufenweise Trocknung von Vorteil sein kann.

Die Verformung des imprägnierten Trägers kann unter Verwendung verschiedener, bekannter Verfahren, wie beispielsweise Extrudieren, Tablettieren oder Granulieren erfolgen. Rohrförmige Formkörper werden üblicherweise durch Extrudieren oder Tablettieren, kugelförmige Formkörper üblicherweise durch Granulieren oder gegebenenfalls durch Tablettieren hergestellt.

Bei der Herstellung von Formkörpern kann der imprägnierte Träger in der Regel mit Wasser und unter Zusatz von Extrudierhilfsmitteln wie Stärke, Methylcellulose, Graphit, Polyethylenglykole, Polyvinylalkohole, Polyvinylpyrrolidone, Polyacrylester, Stearinsäure und deren Metallsalze, Naphthalin, Ammoniak, Ameisensäure, Oxalsäure oder Salpetersäure entweder als porositätsverbesserndes Mittel oder als Gleitmittel oder als Peptisierungsmittel verformt oder zunächst geknetet und anschließend verformt werden. Bevorzugt ist dabei die Knetung und die anschließende Verformung, insbesondere das Extrudieren. Die erhaltenen Formlinge werden dann bei Temperaturen im Bereich von Raumperatur bis ca. 400°C getrocknet bzw. calciniert, wobei auch eine stufenweise Temperaturbehandlung von Vorteil sein kann.

Bei der Herstellung von Formkörpern durch Tablettierverfahren können ein oder mehrere Zusätze, wie z.B. Bindemittel und/oder Schmiermittel (Gleitmittel) verwendet werden. Als Beispiel für solche Zusätze seien Graphit, Stearinsäure und deren Metallsalze wie z.B. Aluminiumstearat oder Magnesiumstearat, Talkum, Methylcellulose bzw. andere modifizierte Cellulosen beispielsweise 1,2-Hydroxymethylcellulose, Glycerinmonostearat, Polyethylenglykole, Polyvinylalkohole, Polyvinylpyrrolidone, Polyacrylester genannt.

Bei der Herstellung von Formkörpern durch Granulieren, können ebenfalls die üblichen, dem Fachmann hinreichend bekannten Granulierhilfsmittel verwendet werden.

Bevorzugt herzustellende Formkörper sind Ringe oder Tabletten, die je nach dem gewünschten Verwendungszweck bzw. dem Einsatzort im Reaktor im Bereich der Abmessungen von

4 -12 mm für den Außendurchmesser

2 -8 mm für den Innendurchmesser (bei Ringen)

3 - 12 mm für die Höhe

liegen.

Die Verwendung der erfindungsgemäß hergestellten Katalysatoren erfolgt zur Herstellung von 1,2-Dichlorethan durch Oxichlorierung von Ethylen. Die Durchführung des Verfahrens kann einstufig oder auch in mehreren Stufen erfolgen. Wird in mehreren Stufen gearbeitet, können einzelne Einsatzstoffe, z.B. Luft/Sauerstoff oder Chlorwasserstoff aufgeteilt und jedem Reaktor getrennt zugeführt werden. Die Umsetzung kann auch in Kreisgasfahrweise durchgeführt werden, indem das aus der Reaktionszone austretende Reaktionsgemisch teilweise mit frischem Ethylen, Chlorwasserstoff und Luft bzw. Sauerstoff vermischt in die Reaktionszone zurückgeführt wird. Gegebenenfalls kann aus dem Reaktionsgemisch vor der Rückführung vollständig oder zum Teil 1,2-Dichlorethan und Wasser abgetrennt werden.

Mindestens einer der Reaktoren, bei Verwendung von 2 Reaktoren vorzugsweise der Erste, bei Verwendung von drei Reaktoren vorzugsweise der Erste oder der Erste und der Zweite, ist in mehrere aufeinanderfolgende Reaktionszonen aufgeteilt, wobei die Katalysatoraktivität in den Reaktionszonen in Produktstromrichtung zunimmt. Die Zahl der Reaktionszonen in den einzelnen Reaktoren sowie die relative Größe der Zonen ist von Fall zu Fall verschieden. Die günstigsten Bedingungen lassen sich durch prinzipiell einfache Optimierungsversuche ermitteln. Die Herstellung von Katalysatorzonen mit abgestufter Aktivität erfolgt durch bekannte Maßnahmen wie Erniedrigung der Kupfer-(II)-chlorid-Konzentration, die Dotierung mit Alkali oder die Verdünnung der Katalysatorschüttung mit Inertmaterial.

Die nachfolgenden Beispiele sollen die Erfindung weiter erläutern:

Herstellung der Katalysatoren:

Beispiel 1

Pulverförmiges $\gamma$-Al$_2$O$_3$ mit einer Korngröße von kleiner als 0,125 mm und einer spezifischen Oberfläche von 200 m$^2$/g wird mit einer wäßrigen Lösung von CuCl$_2 \cdot$2H$_2$O und KCl imprägniert. Die Konzentration von CuCl$_2 \cdot$2H$_2$O und KCl in der Tränklösung wird so gewählt, daß nach dem Trocknen der Katalysator einen Kupfergehalt von 7,2 Gew.-% und einen Kaliumgehalt von 0,82 Gew.-%,

bezogen auf die Katalysatorgesamtmasse, aufweist. Der imprägnierte Träger wird anschließend unter Stickstoff zunächst 10 Stunden bei 80°C und danach je 3 h bei 120°C und 210°C getrocknet.

500 g des imprägnierten Trägers werden anschließend mit 20 g Graphitpulver durchmischt und mit einer Tablettiervorrichtung zu Tabletten mit den Maßen 5 mm Höhe und 5 mm Durchmesser verpreßt.

**Beispiel 2**

500 g des imprägnierten Trägers gemäß Beispiel 1 werden mit 20 g Aluminiumstearat vermischt und zu Tabletten mit 5 mm Höhe und 5 mm Durchmesser verpeßt.

**Beispiel 3**

500 g des imprägnierten Trägers gemäß Beispiel 1 werden mit 20 g Glycerinmonostearat vermischt und zu Tabletten mit 5 mm Höhe und 5 mm Durchmesser verpeßt.

**Beispiel 4**

500 g des imprägnierten Trägers gemäß Beispiel 1 werden mit 20 g Stearinsäure vermischt und zu Tabletten mit 5 mm Höhe und 5 mm Durchmesser verpeßt.

**Beispiel 5**

500 g des imprägnierten Trägers gemäß Beispiel 1 werden mit 20 g Methylcellulose vermischt und zu Tabletten mit 5 mm Höhe und 5 mm Durchmesser verpeßt.

**Beispiel 6**

500 g des imprägnierten Trägers gemäß Beispiel 1 werden mit 20 g Talkum vermischt und zu Tabletten mit 5 mm Höhe und 5 mm Durchmesser verpeßt.

**Beispiel 7**

500 g des imprägnierten Trägers gemäß Beispiel 1 werden mit 20 g Dextrin vermischt und zu Tabletten mit 5 mm Höhe und 5 mm Durchmesser verpeßt.

**Vergleichsbeispiel**

Katalysator, hergestellt nach dem Stand der Technik:

500 g $\gamma$-$Al_2O_3$ Tabletten mit den Abmessungen 5 mm Höhe und 5 mm Durchmesser werden mit einer Lösung imprägniert, die $CuCl_2 \bullet 2H_2O$ und KCl enthält. Die Konzentration an $CuCl_2 \bullet 2H_2O$ und KCl in der Tränklösung wird so gewählt, daß der Katalysator nach dem Trocknen einen Kupferionengehalt von 7,2 Gew.-% und einen Kaliumionengehalt von 0,82 Gew.-% aufweist. Die Trocknung der getränkten Formkörper erfolgt wie bei der erfindungsgemäßen Herstellung.

Prüfung der Katalysatoren

Zur Prüfung der Aktivität der erfindungsgemäß hergestellten Katalysatoren bzw. des Katalysators, hergestellt nach dem Stand der Technik, werden 90 cm³ des Katalysators in einen Edelstahlreaktor mit 64 mm Innendurchmesser eingefüllt und pro Stunde mit einer Gasmischung beaufschlagt, die 0,986 mole Chlorwasserstoff, 0,493 mole Ethylen und 0,246 mole Sauerstoff in Form von Luft enthält. Ein Teil des aus der Katalysatorschüttung austretenden Gases wird mit frischem Einsatzgas vermischt und in die Reaktionszone zurückgeführt. Das Verhältnis von rückgeführtem Reaktionsgas zu frischem Einsatzgas beträgt 20:1.

Die Temperatur in der Katalysatorschicht wird auf 225°C, der Druck im Reaktor auf 1 bar eingestellt. Nach Erreichen eines stationären Betriebszustandes werden dem aus der Reaktionszone austretenden Gas Proben entnommen und daraus der Umsatz und die Ausbeute am 1,2-Dichlorethan bestimmt. Als Maß für den Umsatz dient der prozentuale Anteil des umgesetzten Chlorwasserstoffs, als Maß für die Ausbeute der prozentuale Anteil vom umgesetzten Ethylen, der zu 1,2-Dichlorethan umgesetzt wurde.

Zur Prüfung der Standzeit der erfindungsgemäßen Katalysatoren werden ungefähr 25 g in einen Edelstahlreaktor mit 10 mm Innendurchmesser eingefüllt. Der Druckverlust der Katalysatorschüttung wird bei Raumtemperatur bei einer Beaufschlagung mit 600 l Stickstoff pro Stunde und einem Druck vor der Schüttung von 1,5 m Wassersäule gemessen.

Danach wird der Reaktor mit einer Salzschmelze auf 250°C aufgeheizt und der Katalysator pro Stunde mit einem Gas beaufschlagt, welches 16,9 l Chlorwasserstoff, 15,9 l Ethylen und 32,9 l Luft enthält. Nach 6 Tagen wird der Reaktor unter Stickstoff abgekühlt und der Druckverlust unter den obigen Bedingungen erneut gemessen.

Als Maß für die Standzeit des Katalysators wird der prozentuale Anstieg des Druckverlustes angegeben.

Ergebnisse

Die Prüfungsergebnisse der nachfolgenden Tabelle zeigen, daß bei der Herstellung der Katalysatoren nach dem erfindungsgemäßen Verfahren Vorteile resultieren. Sowohl die Aktivität als auch die Selektivität ist bei der erfindungsgemäßen Herstellung besser, wobei sich auch bei der Standzeit Verbesserungen im Vergleich zur Herstellung nach dem Stand der Technik ergeben.

| Beispiel | HCl-Umsatz [%] | 1,2-Dichlorethan-Selektivität [%] | Anstieg des Druckverlustes [%] |
|---|---|---|---|
| 1 | 77,7 | 97,8 | 18 |
| 2 | 75,4 | 97,0 | 33 |
| 3 | 77,8 | 98,0 | 25 |
| 4 | 78,9 | 97,3 | 35 |
| 5 | 79,9 | 97,9 | 32 |
| 6 | 79,2 | 98,0 | 17 |
| 7 | 78,3 | 98,0 | 27 |
| Katalysator nach dem Stand der Technik | 75,3 | 96,8 | 40 |

**Ansprüche**

1. Verfahren zur Herstellung eines geformten, Kupfer-(II)-chlorid und/oder Kupferoxichlorid enthaltenden Trägerkatalysators, dadurch gekennzeichnet, daß man

(a) zuerst die Oberfläche eines porösen Trägermaterials mit 2 bis 15 Gew.-% Kupferionen in Form von Kupfer-(II)-chlorid und/oder Kupferoxichlorid und 0 bis 5 Gew.-% Alkalimetallionen, jeweils bezogen auf das Gesamtgewicht des Katalysators, imprägniert und

(b) das so imprägnierte Trägermaterial zu Katalysatorformkörpern formt.

2. Verfahren nach Anspruch 1, dadurch gekennzeichnet, daß man 3 bis 10 Gew.-% Kupferionen in Form von Kupfer-(II)-chlorid und/oder Kupferoxichlorid und 0,2 bis 3 Gew.-% Alkalimetallionen verwendet.

3. Verfahren nach Ansprüchen 1 oder 2, dadurch gekennzeichnet, daß man Alkalimetallchloride verwendet.

4. Verfahren nach einem der Ansprüche 1 bis 3, dadurch gekennzeichnet, daß man das imprägnierte Trägermaterial mit Wasser anteigt und, gegebenenfalls unter Zusatz eines Extrudierhilfsmittels, zu Formkörpern extrudiert.

5. Verfahren nach einem der Ansprüche 1 bis 3, dadurch gekennzeichnet, daß man das imprägnierte Trägermaterial, gegebenenfalls unter Zusatz von Gleit-und/oder Bindemitteln, tablettiert.

6. Verfahren nach einem der Ansprüche 1 bis 3, dadurch gekennzeichnet, daß man das imprägnierte Trägermaterial, gegebenenfalls unter Zusatz von Granulierhilfsmitteln, granuliert.

7. Katalysator, insbesondere für die Oxichlorierungsreaktion, erhältlich nach einem der Ansprüche 1 bis 6.

8. Verwendung des Katalysators nach Anspruch 7 als Oxichlorierungskatalysator im Katalysatorfestbett.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-1 554 095 (SHELL) <br><br> * Seite 3, linke Spalte * <br><br>--- | | B 01 J 23/78 <br> B 01 J 27/10 // <br> C 07 C 17/156 |
| A | FR-A-2 368 454 (DIAMOND SHAMROCK) <br> * Seite 4 * <br><br>--- | | |
| A | EP-A-0 062 320 (KANEGAFUCHI KAGAKU KOGYO K.K.) <br><br>--- | | |
| A | EP-A-0 041 330 (PPG INDUSTRIES) <br><br>----- | | |

| RECHERCHIERTE SACHGEBIETE (Int. Cl.4) |
|---|
| B 01 J 23/00 <br> B 01 J 27/00 |

Der vorliegende Recherchenbericht wurde für alle Patentansprüche erstellt.

| Recherchenort | Abschlußdatum der Recherche | Prüfer |
|---|---|---|
| DEN HAAG | 03-10-1986 | DEVISME F.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument